(19) Europäisches Patentamt
European Patent Office
Office européen des brevets

(11) Veröffentlichungsnummer: **0 403 968 A2**

# (12) EUROPÄISCHE PATENTANMELDUNG

(21) Anmeldenummer: **90111306.8**

(22) Anmeldetag: **15.06.90**

(51) Int. Cl.⁵: **C08G 63/692, A61L 27/00, A41G 3/00**

(30) Priorität: **20.06.89 DE 8907506 U**

(43) Veröffentlichungstag der Anmeldung: **27.12.90 Patentblatt 90/52**

(84) Benannte Vertragsstaaten: **AT BE CH DE DK ES FR GB IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Striegl, Peter**
**Greifstrasse 16**
**D-8903 Bobingen(DE)**

## (54) Kunsthaar aus einem flammfesten Polyester-Monofil.

(57) Beschrieben wird Kunsthaar aus einem flammfesten Monofil aus Polyestern aus Dicarbonsäure- und Diolkomponenten in die ein flammhemmendes Mittel inkorporiert ist.

Das flammhemmende Mittel besteht aus in die Polyesterketten einkondensierten Kettengliedern mit den Struktureinheiten der allgemeinen Formel

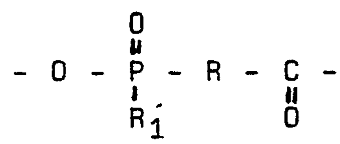

wobei R einen Alkylen-, Arylen- oder einen Aralkylenrest und $R_1$ einen Alkyl-, Aryl- oder Aralkylrest bedeuten.

***Fig. 1***

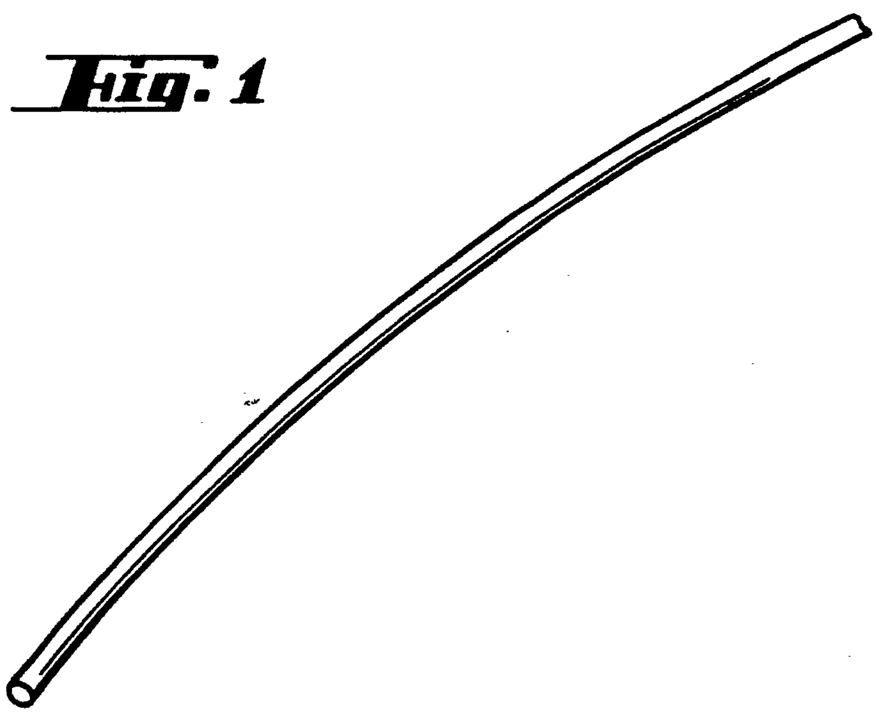

EP 0 403 968 A2

# Kunsthaar aus einem flammfesten Polyester-Monofil

Die Erfindung betrifft ein Kunsthaar aus einem flammfesten Monofil aus linearen Polyestern.

Da es für die Haarersatzhersteller immer schwieriger wird, Naturhaare zu beschaffen, gewinnen Kunsthaare immer mehr an Bedeutung. Das Kunsthaar für Perücken, Toupees und Haarersatzteile bzw. Prothesen, und zwar sowohl für Menschen wie auch für Spielzeug (Puppen etc.), besteht heute insbesondere aus Modacryl oder Polyester-Monofilen. Diese Materialien sind alle brennbar.

Aus den JA-Anmeldungen 73 06 616, 75010 413, 47 14 416 und 74 044 009 sind Kunsthaare aus Polyestern bekannt geworden, die eine Halogen-Verbindung aus Flammschutzmittel enthalten. Halogenverbindungen können aber unter thermischer Belastung zu toxischen Verbindungen führen und sind deshalb für den Einsatz im menschlichen Bereich bedenklich. Aus der JA-Anmeldung 61 194 216 sind tierhaarähnliche Polyesterfasern bekannt, die eine Phosphor-Verbindung als Flammschutz enthalten. Auch diese Fasern sind zum Einsatz beim Menschen wenig geeignet. Die DE-OS 21 08 551 schließlich erwähnt ebenfalls, Kunsthaare flammfest auszurüsten, ohne jedoch Angaben eines geeigneten Lösungsweges zu machen.

Der Erfindung liegt die Aufgabe zugrunde, ein Kunsthaar aus einem Monofil aus linearen Polyestern zu schaffen, das bei einfacher Herstellbarkeit gute flammhemmende Eigenschaften besitzt, ohne hierdurch die Gefahr toxischer Nebenwirkungen hervorzurufen.

Diese Aufgabe wird bei einem Kunsthaar der eingangs angegebenen Gattung erfindungsgemäß dadurch gelöst, daß das Monofil aus Polyestern aus Dicarbonsäure- und Diolkomponenten besteht, in die ein flammhemmendes Mittel inkorporiert ist.

Bevorzugt besteht das flammhemmende Mittel aus in die Polyesterketten einkondensierten Kettengliedern mit den Struktureinheiten der allgemeinen Formel

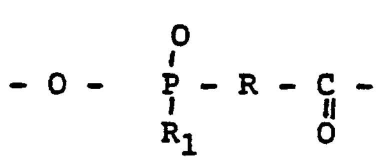

wobei R einen gesättigten offenkettigen oder zyklischen Alkylen-, einen Arylen- oder einen Aralkylenrest und $R_1$ einen alkylrest mit bis zu 6 C-atomen, einen Aryl- oder Aralkylrest bedeuten.

Derartige durch Rohstoffmodifikation flammfest gemachte Polyester sind aus der DE-PS 23 46 787 bekannt. Dort werden als Anwendung jedoch nur Fasern und Fäden für Textilien und technische Artikel wie Planen, Stoffe, Teppiche und Gardinen erwähnt.

Durch die Erfindung wird dagegen erstmals die Verwendung von Monofilen aus flammfest modifizierten Polyestern für Kunsthaare vorgeschlagen. Auf diese Weise ist es gelungen, derartige Kunsthaare in einfacher Weise flammfest zu machen, ohne daß hierzu aufwendige Ausrüstungen erforderlich sind, die toxische Nebenwirkungen aufweisen können.

Um allen Anforderungen, die an Kunsthaare hinsichtlich Mattigkeit gestellt werden, gerecht zu werden, können über Zudosierungen eines Mattierungsmittels verschiedene Mattierungsgrade eingestellt werden. Eine dem Menschenhaar angepaßte Brillanz des Haares läßt sich durch eine spezielle Querschnittsform des Monofils erreichen.

Das erfindungsgemäß ausgebildete Kunsthaar weist neben den flammhemmenden Eigenschaften hervorragende thermische Licht-und Feuchtigkeits-Beständigkeit auf. Ein weiterer Vorteil besteht darin, daß das erfindungsgemäß ausgebildete Haar im Gegensatz zum menschlichen Haar mikrobenbeständig und somit zur Dauerbefestigung auf dem Kopf geeignet ist.

Anhand der Zeichnung wird ein Ausführungsbeispiel der Erfindung erläutert. Es zeigt:

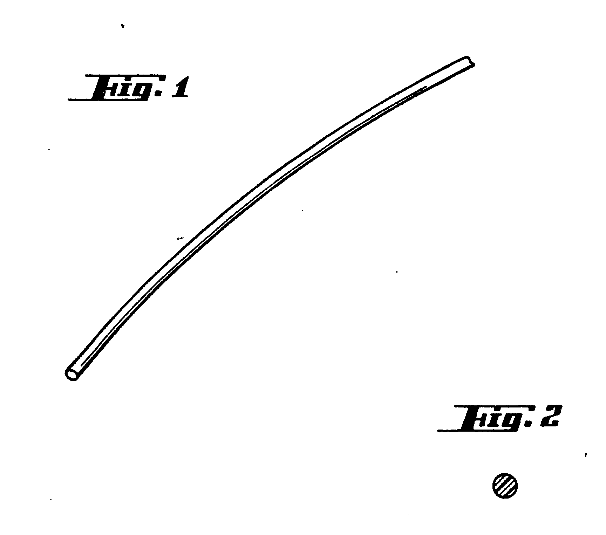

Fig. 1 eine schematische Darstellung eines Kunsthaares in vergrößertem Maßstab;

Fig. 2 einen Querschnitt des Kunsthaares in Fig. 1.

Das in der Zeichnung dargestellte Kunsthaar besteht aus einem Monofil aus linearen Polyestern, die durch eine Rohstoffmodifikation gemäß der DE-PS 23 46 787 flammfest gemacht worden ist.

Wie in Fig. 2 gezeigt, hat das Kunsthaar einen kreisrunden Querschnitt. Der Durchmesser des Kunsthaares liegt im Bereich von 0,050 bis 0,10 mm. Die Durchmessergröße richtet sich hauptsächiich nach der Menschenrasse. Die Haare europäischer Menschen sind dünner als die indischer Menschen, und die Haare indischer Menschen sind wiederum dünner als die asiatischer Menschen.

Statt eines kreisrunden Querschnittes kann das Kunsthaar auch einen elliptischen, hantel- oder knochenförmigen, oder anderen unrunden Querschnitt haben. Hierdurch läßt sich eine dem Menschenhaar angepaßte Brillanz des Haares erreichen.

## Ansprüche

1. Kunsthaar aus einem flammfesten Monofil

aus linearen Polyestern, dadurch **gekennzeichnet,** daß das Monofil aus Polyestern aus Dicarbonsäure- und Diolkomponenten besteht, in die ein flammhemmendes Mittel inkorporiert ist.

2. Kunsthaar nach Anspruch 1, dadurch **gekennzeichnet,** daß das flammhemmende Mittel aus in die Polyesterketten einkondensierten Kettengliedern mit den Struktureinheiten der allgemeinen Formel

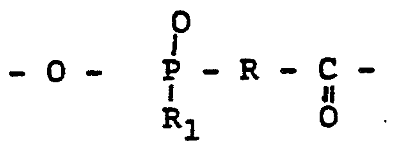

besteht, wobei R einen gesättigten offenkettigen oder zyklischen Alkylen-, einen Arylen- oder einen Aralkylenrest und $R_1$ einen Alkylrest mit bis zu 6C-Atomen, einen Aryl- oder Aralkylrest bedeuten.

3. Kunsthaar nach Anspruch 1 oder 2, dadurch **gekennzeichnet,** daß das monofil bei rundem Querschnitt einen Durchmesser von 0,050 - 0,10 mm hat.

4. Kunsthaar nach Anspruch 1 oder 2, dadurch **gekennzeichnet,** daß das Monofil einen hantel- oder knochenförmigen Querschnitt hat.

Fig. 1

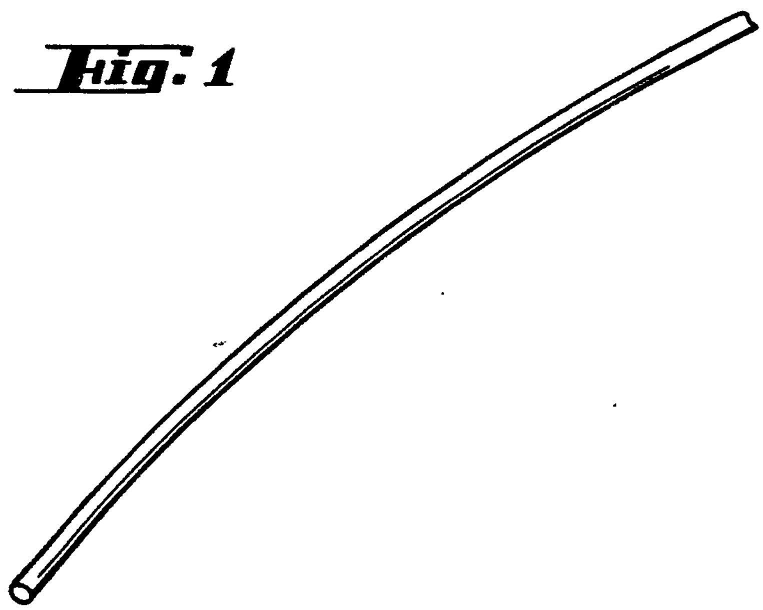

Fig. 2